# EUROPÄISCHE PATENTSCHRIFT

(11) **EP 0 608 737 B1**
(45) Veröffentlichungstag und Bekanntmachung des Hinweises auf die Patenterteilung: **21.08.2002**
(21) Anmeldenummer: 94100564.7
(22) Anmeldetag: 17.01.1994
(51) Int. Cl.: C07H 21/00, G01N 33/53

(54) **Sulfocumarinhaltige Nukleotide und deren Verwendung in Nachweisverfahren für Nukleinsäuren**
Sulpho-cumarin containing nucleotiden and their use in detection methods for nucleic acids
Nucléotides qui contiennent sulfocumarin et leur utilisation dans les méthodes de détection des acides nucléiques

(30) Priorität: 29.01.1993 DE 4302459
(43) Veröffentlichungstag der Anmeldung: 03.08.1994
(73) Patentinhaber: BAYER AG, 51368 Leverkusen (DE)
(72) Erfinder: Köcher, Jürgen, Dr., D-40764 Langenfeld (DE); Springer, Wolfgang, Dr., D-42113 Wuppertal (DE); Kuckert, Eberhard, Dr., D-51375 Leverkusen (DE); Böcker, Thomas, Dr., D-42799 Leichlingen (DE)

(56) Entgegenhaltungen:
- EP-A- 0 157 384
- EP-A- 0 513 560
- EP-A- 0 527 433
- WO-A-91/14697
- IEEE JOURNAL OF QUANTUM ELECTRONICS Bd. 26, Nr. 12 , 1990 , USA Seiten 2158 - 2161 S.V.CHEKALIN ET AL. 'Laser Femtosecond MPI Mass Spectrometry of Dye-Labeled Nucleotides.'

## Beschreibung

Die vorliegende Erfindung betrifft die Herstellung von Nukleotiden mit einem fluoreszierenden Cumarinrest als Substituenten, den enzymatischen Einbau dieser Nukleotide in Nukleinsäuren und den Nachweis von Nukleinsäuren definierter Sequenz durch Hybridisierung mit einer komplementären, cumarinmarkierten Nukleinsäure (Gensonde).

Eine der meist angewandten molekularbiologischen Techniken ist die DNA/DNA-, RNA/RNA- bzw. RNA/DNA-Hybridisierung zum Nachweis homologer Nukleinsäuresequenzen. Dabei wird eine als Sonde verwendete Nukleinsäure (DNA oder RNA) markiert und mit einer zu untersuchenden Nukleinsäure (DNA oder RNA) unter Hybridisierungsbedingungen in Kontakt gebracht. Bei vorhandener Homologie zwischen der als Sonde verwendeten Nukleinsäure und der nachzuweisenden Nukleinsäure kommt es zur Hybridisierung der jeweils komplementären Nukleinsäureeinzelstränge unter Ausbildung eines Hybriddoppelstranges. Anschließend werden die Hybride nachgewiesen.

In der Vergangenheit wurden die als Sonden verwendeten Nukleinsäuren meist durch den Einbau radioaktiv derivatisierter Ribo- oder Desoxyribonukleosidtriphosphate markiert, und die Hybride durch Autoradiographie nachgewiesen.

Diese Technik der Gensondenmarkierung hat sich als besonders empfindliche Methode bewährt, ist jedoch auch problematisch aufgrund der Handhabung radioaktiver Materialien. So werden an die Laborsicherheit und die Entsorgung radioaktiver Verbindungen besondere Anforderungen gestellt

Weiterhin sind radioaktive Materialien aufgrund ihrer Halbwertszeit nur einen begrenzten Zeitraum verwendbar.

Deswegen sind in der Vergangenheit schon einige nichtradioaktive Markierungsverfahren entwickelt worden. Die Gensonden werden hierbei beispielsweise mit Biotinmolekülen (EP 0 063 879) oder Digoxigeninmolekülen (EP 0 324 474 A1) markiert, wobei der Einbau dieser Nachweismoleküle in eine Nukleinsäuresonde auf chemischem, photochemischem oder enzymatischem Weg erfolgen kann. Nach Derivatisierung der Nukleinsäuresonde erfolgt die Hybridisierung mit der nachzuweisenden Nukleinsäuresequenz. Der Nachweis der Hybride erfolgt durch Bindung eines (Strept)-Avidin-Markerenzymkonujugates an Biotin oder durch Bindung eines Antidigoxigeninantikörper-Markerenzymkonjugates an Digoxigenin.

Nachteile dieser Methoden bestehen darin, daß die an die Nukleinsäuresonde gebundenen Markermoleküle nicht direkt nachweisbar und somit quantifizierbar sind. Vielmehr müssen diese Markermoleküle wie Biotin oder Digoxigenin durch die Bindung an solche Moleküle nachgewiesen werden, die mit den Markermolekülen spezifische Wechselwirkungen eingehen. Diese Moleküle, wie beispielsweise Streptavidin für den Nachweis von Biotin, können ihrerseits nicht direkt nachgewiesen werden, sondern müssen in geeigneter Weise mit einer Markersubstanz versehen sein. Das kann beispielsweise ein Fluoreszenzfarbstoff sein, dessen Konzentration durch Messung der Fluoreszenz bestimmt werden kann. Sehr häufig ist diese Markersubstanz jedoch ein Enzym, das ein zugegebenes Substrat in eine quantitativ auswertbare Form überführt. Ein solches Substrat kann beispielsweise die Vorstufe eines Farbstoffes, eines Fluoreszenzfarbstoffes oder einer chemilumineszenten Verbindung sein.

Damit ergibt sich, daß zum Nachweis eines Hybrides durch eine markierte Gensonde weitere Verfahrensschritte notwendig sind. Jeder zusätzliche Verfahrensschritt birgt die Gefahr von Fehlern oder Ungenauigkeiten. Die zur Bindung an beispielsweise Biotin oder Digoxigenin benötigten Verbindungen sind Biomoleküle, wie Streptavidin oder Antikörper, deren Herstellung aufwendig ist. Diese Biomoleküle müssen noch mit zusätzlichen Markerenzymen gekoppelt werden. Die immunologischen und enzymatischen Reaktionen sind mit vielen Verfahrensschritten verbunden, die die Auswertung der Hybridisierungsreaktion sehr kompliziert machen.

EP-A-0 527 433 beschreibt Cumarinderivate, die zur Fluoreszenzmarkierung von Nukleinsäuren verwendet werden können. Die dort beschriebenen Substanzen enthalten jedoch keinen Sulfo-Substituenten.

Deshalb besteht nach wie vor ein Bedarf an Substanzen für die Hybridisierung von Nukleinsäuresonden, die leicht verfügbar sind, einfach an Nukleinsäuren gebunden werden können und mit geringem Aufwand nachweisbar sind.

Ziel der Erfindung war es, Markierungssubstanzen für Nukleinsäuren zu entwickeln, die diese Voraussetzungen erfüllen.

Es wurde gefunden, daß fluoreszierende Nukleotide der Formel

A - B - C (I)

auf enzymatischem Weg in Nukleinsäuren eingebaut werden können und intensive und gut nachweisbare Markierungen ergeben,
wobei
- A: ein natürliches oder synthetisches Nukleotid oder ein Derivat hiervon.
- B: ein Brückenglied mit zwei verknüpfbaren Zentren
und
- C: ein Cumarinrest der Formel 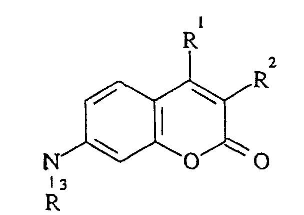 bedeutet, wobei
- R¹: Wasserstoff oder Cyano ist,
- R²: Phenyl oder Thiazolyl, das in 2-, 4- oder 5-Position gebunden ist, wobei beide Reste einen SO₃H-Rest tragen sowie weiter substituiert sein können,
oder wobei der Thiazolylrest in 4- oder 5-Position mit einem Benzolring kondensiert ist, der mit einer Sulfogruppe substituiert ist und gegebenenfalls zusätzlich substituiert ist.
- R³: H, C₁₋₄-Alkyl, C₁₋₄-Alkoxycarbonyl-C₁₋₄-alkyl, oder Phenylsulfonyl bedeutet, wobei C₁₋₄-Alkyl unsubstituiert oder mit Hydroxyl, Amino, Carboxyl, C₁₋₄-Alkoxycarbonyl oder Sulfo substituiert sein kann, und wobei Phenylsulfonyl unsubstituiert oder einfach oder mehrfach durch Chlor, Brom, C₁₋₄-Alkyl oder Sulfo substituiert sein kann.
- R² oder R³: kann durch eine primäre oder sekundäre Aminogruppe, Hydroxyl, Carboxyl oder C₁₋₄-Alkoxylcarbonyl substituiert sein oder selbst einen solchen Substituenten darstellen, oder R² oder R³ kann durch Hydrolyse in eine solche Gruppe umgewandelt werden,

Bevorzugt sind Verbindungen der Formel (I), wobei
- A: einen Rest darstellt, der aus der Gruppe von natürlichen oder synthetischen Nukleotiden ausgewählt wird, die aus AMP, ADP, ATP, GMP, GDP, GTP, CMP, CDP, CTP, UMP, UDP, UTP, TMP, TDP, TTP, 2-Me-AMP, 2-Me-ADP, 2-Me-ATP, 1-Me-GMP, 1-Me-GDP, 1-Me-GTP, 5-Me-CMP, 5-Me-CDP, 5-Me-CTP, 5-MeO-CMP, 5-MeO-CDP, 5-MeO-CTP, Deoxy- oder Dideoxynukleotide aus dieser Reihe, sowie weitere Derivate hiervon, besteht,
- B: ein bifunktionelles Brückenglied mit bis zu 50 Atomen Kettenlänge ist, wobei die Atome C, H, O, N oder S sein können. Dieses Brückenglied kann linear oder auch verzweigt sein,
und
C einen Cumarinrest der Formel 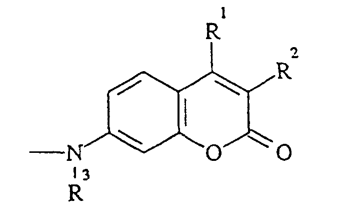 darstellt, in dem
R¹ die obengenannten Bedeutungen besitzt,
R² Phenyl oder Thiazolyl, in 2-, 4- oder 5-Position gebunden, bedeutet, wobei Phenyl mit einer Sulfogruppe substituiert ist und zusätzlich mit Carboxy, C₁₋₄-Alkylcarbonyloxy, Amino, -NH-C₁₋₄-Alkyl, -(CH₂)₁₋₄-NH₂, C₁₋₄-Alkyl, Cyano, Fluor, Chlor, Brom oder Sulfo substituiert ist und wobei der Thiazolylrest mit einer Sulfogruppe substituiert ist und gegebenenfalls noch zusätzliche Substituenten tragen kann,
oder wobei der Thiazolylrest in 4- und 5-Position mit einem Benzolring kondensiert ist, der mit einer Sulfogruppe substituiert ist sowie zusätzlich substituiert sein kann, und
R³ Wasserstoff, Methyl, Ethyl, -(CH₂)₁₋₄-OH, -(CH₂)₁₋₄-NH₂ oder -(CH₂)₁₋₄-COOH oder (CH₂)₁₋₄-SO₃H ist.

Ganz besonders bevorzugt sind Verbindungen der Formel (I), wobei
- A: die obengenannten Bedeutungen hat,
- B: ein bifunktionelles Brückenglied mit 2 bis 20 Atomen aus der Reihe von C, H, O, N oder S ist. Solche Brückenglieder können Peptidderivate, Kohlenwasserstoffe wie Alkylen, Alkenylen, Alkynylen, Arylen oder substituierte Derivate hiervon, Polyalkohole, Polyalkoxide, Polyether, Polyamine, Polyimine, Carbohydrate, -CH=CH-CH₂NH-, -Glycyl-Glycyl-Glycyl-, -NH(CH₂)₅CO-, Spermin oder Spermidin, NH-(CH₂)₆-NH-, -NH-CH₂CH₂-NH-, -CH=CH-CH₂-NH-CO-(CH₂)₅-NH-CO- oder -CH=CH-CH₂-NH-CO-(CH₂)₅-NH-CO-(CH₂)₃- sein,
und
- C: ein Cumarinrest ist mit der oben angegebenen Formel, wobei
R¹ die oben angegebene Bedeutung hat,
R² ein Phenyl oder Thiazolyl, in 2-Position gebunden, ist,
wobei Phenyl mit Sulfo substituiert ist und zusätzlich mit para-Carboxy, para-Amino, para-NH-C₁₋₄-Alkyl, para-CH₂-NH₂, Cyano, Methyl oder Ethyl substituiert ist, und wobei Thiazolyl mit Sulfo substituiert ist und zusätzlich mit Chlor, Cyano oder Carboxyl substituiert ist, oder der Thiazolylrest in 4- und 5-Stellung mit einem Benzolring kondensiert ist, der mit Sulfo substituiert ist und zusätzlich Carboxy oder Amino als Substituenten enthalten kann, und
R³ die oben angegebenen Bedeutungen hat.

Die Verwendung von Cumarinen als Fluoreszenzfarbstoff ist vorteilhaft gegenüber der Verwendung von beispielsweise Fluoreszein, weil sich Cumarine durch besonders hohe Stabilität und Lichtechtheit auszeichnen.

Die Herstellung der erfindungsgemäßen Substanzen erfolgt in der Regel dadurch, daß man eine Cumarinfarbstoffkomponente und eine Nukleotidkomponente miteinander verknüpft. Beide Komponenten enthalten reaktive Zentren, die chemisch derivatisierbar und miteinander verknüpfbar sind. Häufig befinden sich solche Zentren in Seitenketten beider Komponenten. Nach dem Verknüpfen beider Komponenten über diese reaktive Zentren in den Seitenketten erhält man die erfindunggemäßen Verbindungen, und die verknüpften Seitenketten des Nukleotid- und Cumarinteils bilden das Brückenglied B der Formel (I).

Als reaktive und derivatisierbare Zentren sind beispielsweise Hydroxy-, Amino- oder Carboxygruppen verwendbar. Durch Verwendung von beispielsweise Methansulfonsäurechlorid, N,N'-Carbonyldiimidazol oder N-Hydroxysuccinimid können Hydroxyund Carboxygruppen zu reaktiven Estern umgesetzt werden, die mit O- oder N-Nukleophilen unter Ausbildung von Carbonestern oder Carbonamiden verknüpft werden können.

Mit einer reaktiven Seitenkette modifizierte Nukleotide sind nach in der Literatur beschriebenen Verfahren verfügbar, siehe z.B. D. Bergstrom et al., Synlett 1992, 179. Besonders ausführlich beschrieben sind Uridin-5'-triphosphat- und Deoxyuridin-5-triphosphatderivate mit einer Allylaminseitenkette in 5-Position (P.R. Langer et al., Proc. Natl. Acad. Sci. USA, 78, 6633 (1981) oder mit einer Propargylaminseitenkette (F.W. Hobbs, J. Org. Chem. 54, 3420 (1989)) sowie Adenosin-5-triphosphatderivate mit Hexamethylendiaminseitenketten in 6- und 8-Position [V. Folsom et al., Anal. Biochem. 182, 309 (1989); C.-Y. Lee et al., Arch. Biochem. Biophys. 178, 8 (1977)].

Einige dieser Verbindungen sind mittlerweile auch kommerziell erhältlich. Cumarinfarbstoffe sind ebenfalls aus der Literatur bekannt, beispielsweise aus EP-A-0428 939. Diese Produkte sind mit Seitenketten und daran befindlichen reaktiven Zentren erhältlich, die mit der Seitenkette eines modifizierten Nukleotides verknüpfbar sind. So kann ein Cumarinfarbstoff mit einem Carboxysubstituenten in einen reaktiven Carbonsäureester, beispielsweise mit N-Hydroxysuccinimid, überführt werden, der dann mit einer Aminogruppe eines der obengenannten modifizierten Nukleotide zum Carbonamid reagiert.

Ein Problem bei diesen Verknüpfungsreaktionen ist der Unterschied in der Löslichkeit von Nukleotid- und Cumarinkomponente. Nukleotide sind praktisch ausschließlich in Wasser löslich, während Cumarine in H₂O normalerweise unlöslich oder nur sehr schwerlöslich sind. In vielen organischen Lösungsmitteln sind Cumarine ebenfalls nur sehr schlecht löslich.

Die oben beschriebene Verknüpfungsreaktion ist bei zwei in ihrer Löslichkeit so unterschiedlichen Verbindungen nur unter Schwierigkeiten möglich. Man benötigt Lösungsmittelgemische (Wasser und organisches Lösungsmittel), deren Mischungsverhältnis genau ermittelt werden muß, um beide Reaktionspartner in ausreichender Menge in Lösung zu halten und somit eine befriedigende Ausbeute an Reaktionsprodukt zu erhalten.

Die erfindungsgemäßen, cumarinsubstituierten Nukleotide der Formel (I) enthalten einen Cumarinfarbstoff mit mindestens einer Sulfonsäuregruppe. Die Verknüpfung von Nukleotid- und Cumarinkomponente kann daher in wäßriger Lösung vorgenommen werden, weil die Sulfonsäuregruppen die Cumarinmoleküle ausreichend wasserlöslich machen. Dadurch sind die erfindungsgemäßen Produkte der Formel (I) wesentlich einfacher erhältlich als vergleichbare Produkte ohne Suifonsäuregruppen. Die Synthese sulfonierter Cumarine kann dadurch erfolgen, daß man während der Synthese geeignete, mit Sulfogruppen substituierte Vorprodukte verwendet.

Bevorzugt werden jedoch sulfonierte Cumarine dadurch hergestellt, daß man Cumarine nach ihrer Synthese nachträglich durch geeignete Sulfonierungsverfahren derivatisiert. Als Sulfonierungsmittel geeignete Reagentien sind konzentrierte Schwefelsäure mit einem Schwefelsäuregehalt von 70 % und mehr, Monohydrat, Chlorsulfonsäure und Oleum mit Konzentrationen bis zu 65 %.

Die Cumarine werden zu den sulfonierten Derivaten umgesetzt, indem man die Farbstoffe in eines der obengenannten Sulfonierungsreagentien einträgt, bevorzugt bei 0°C bis 50°C, und solange ebenfalls bevorzugt bei 0°C bis 50°C rührt, bis das Ausgangsmaterial vollständig umgesetzt ist. Das Produkt kann nach bekannten Methoden isoliert und gereinigt werden.

Ein weiterer Gegenstand dieser Erfindung ist die Verwendung der mit Sulfocumarinen substituierten Nukleotide der Formel (I) zum Nachweis von Nukleinsäuren definierter Sequenz. Hierzu werden Nukleinsäuren durch den enzymatischen Einbau der mit Cumarinen substituierten Nukleotide derivatisiert. Die erhaltenen fluoreszierenden Nukleinsäuren können als Gensonden zum Nachweis homologer Nukleinsäuresequenzen durch Hybridisierung verwendet werden. Die sich bildenden fluoreszierenden Hybride können einfach nachgewiesen werden.

Eine weitere Anwendungsmöglichkeit der erfindungsgemäßen fluoreszierenden Nukleotide der Formel (I) ergibt sich durch die Möglichkeit, diese Moleküle während enzymatischer Amplifikationsverfahren in Nukleinsäuren einzubauen. Die durch spezifische Vervielfältigung der zu analysierenden Nukleinsäure in Gegenwart der erfindungsgemäßen fluoreszierenden Nukleotide entstehenden Nukleinsäurestücke enthalten an Stelle von natürlichen Nukleotiden auch diese fluoreszierenden Nukleotide der Formel (I). Hierdurch werden die durch Amplifikation hergestellten Nukleinsäurestücke fluoreszent, so daß ihre Bildung leicht und direkt nachgewiesen werden kann. Die entstandenen fluoreszierenden Nukleinsäurestücke können ebenfalls als markierte Gensonden für Hybridisierungsversuche eingesetzt werden.

Nukleinsäuren (DNA und RNA) können durch verschiedene enzymatische Methoden mit fluoreszenten Nukleotiden derivatisiert werden. Die "Random-primed"-Methode (Anal. Biochem. 132, 6 (1983)) ist eine Methode zur Derivatisierung von DNA und beruhrt auf der Hybridisierung einer Mischung aller möglichen Hexanukleotidsequenzen mit der zu modifizierenden DNA. Ausgehend von den 3'-OH-Enden dieser Hexanukleotide wird durch DNA-Polymerasen wie das Klenow-Enzym oder anderer DNA-Polymerasen der zum Einzelstrang komplementäre Strang synthetisiert. In den komplementären Strang werden vier als Substrat der DNA-Polymerasen angebotene Desoxyribonukleotide eingebaut. Wird mindestens eins dieser Desoxyribonukleotide durch ein cumarinsubstituiertes Nukleotid ersetzt, erhält man komplementäre DNA, die mit Fluoreszenzfarbstoffen markiert ist.

Anstelle einer Mischung von kurzen Oligodesoxyribonukleotiden mit den unterschiedlichsten Sequenzen kann man auch Oligodesoxyribonukleotide mit spezifischen Sequenzen ("Specific Primer") verwenden. Diese "Specific Primer" binden einheitlich nur an den komplementären Abschnitt einer Einzelstrang-DNA, und die Synthese der komplementären DNA beginnt nur ausgehend von 3'-OH-Ende dieser spezifischen Primer. Wie bei der "Random-Primed"-Methode erfolgt auch hier die Markierung der komplementären DNA dadurch, daß man den DNA-Polymerasen mindestens ein Nukleotid anbietet, das einen Cumarinfarbstoff enthält

Die Methode der "Nick-Translation" (J.Mol.Biol. 113, 237 (1977) beruht auf der Einwirkung einer kleinen Menge des Enzyms DNase I auf doppelsträngige DNA. Die DNase I erzeugt Einzelstrangbrüche in der doppelsträngigen DNA. Gleichzeitig sind E.coli DNA Polymerase I sowie die als Substrate dieses Enzyms dienenden 4 Desoxyribonukleotide in der Reaktionsmischung anwesend. Die E.coli DNA Polymerase I baut an den Einzelstrangbrüchen die 5'-endständigen Desoxyribonukleoside ab, und gleichzeitig an das benachbarte freie 3'-OH-Ende eines der als Substrat angebotenen Desoxyribonukleotide ein. Der Einzelstrangbruch wandert durch wiederholtes Ablaufen dieses Vorganges zum 3'-Ende. Ersetzt man mindestens eines der vier als Substrat angebotenen Nukleotide durch ein cumarinsubstituiertes Nukleotid, erhält man durch Anwendung dieser "Nick-Translationsmethode" fluoreszierende DNA.

Beim 3'-End Labeling von doppel- oder einzelsträngiger DNA macht man sich das Enzym Terminale Transferase zu Nutze, welches Desoxyribonukleotide oder Ribonukleotide an das 3'-OH-Ende anknüpft. Dieses Enzym benötigt mindestens eine Art der Desoxyribo- oder Ribonukleotide als Substrat. Anstelle eines natürlichen Nukleotides kann man auch mit Cumarinen substituierte Desoxyribo- oder Ribonukleotide als Enzymsubstrat einsetzen. Die am 3'-OH-Ende verlängerte Nukleinsäure enthält dann diese Verbindungen und wird dadurch fluoreszierend.

Die Methode der "Reversen Transkription" überführt einzelsträngige Ribonukleinsäure oder doppelsträngige Ribonukleinsäure nach Überführung in die Einzelstränge in die entsprechende DNA. Dazu werden Oligodesoxyribonukleotide als Primer an die komplementären Abschnitte der RNA angelagert. Mit Hilfe des Enzyms Reverse Transkriptase wird, ausgehend von 3'-OH-Ende des Primers, der zum RNA-Strang komplementäre DNA-Strang synthetisiert. Bei dieser DNA-Synthese werden vier Arten von Desoxyribonukleotiden als Enzymsubstrat angeboten, wobei mindestens eins ein cumarinsubstituiertes Derivat ist. Die Reverse Transkriptase baut auch dieses Material in den neu gebildeten DNA-Strang ein, worauf dieser mit Fluoreszenzfarbstoffen markiert ist.

Eine weitere Möglichkeit, Nukleinsäuren mit den erfindungsgemäßen cumarinsubstituierten Nukleotiden herzustellen, ergibt sich durch die Verwendung von Enzymen, die aus einer DNA-Matrize RNA herstellen. Solche Enzyme sind Phagencodierte RNA-Polymerasen wie SP6-, T3- oder T7-RNA Polymerasen. Diese Enzyme benötigen doppelsträngige DNA, die SP6-, T3- oder T7-Promotoren enthalten, und vier Ribonukleotide als Substrate für die RNA-Synthese (J. Mol.Biol. 166, 477 (1983)).

Die Promotorregionen können z.B. in Transkriptionsvektoren insertiert sein, eine andere Möglichkeit ist die Verwendung von kurzen, einzelsträngigen Nukleinsäuren, die so konstruiert sind, daß sie durch Bildung von Haarnadelschleifen ("hairpins") die für die RNA-Polymerasen benötigten doppelsträngigen Promotorregionen enthalten (EP 4 27 073 und EP 4 27 074). Durch Verwendung von Ribonukleotiden als Substrate, die mit Cumarinfarbstoffen substituiert sind, erhält man RNA-Moleküle mit Fluoreszenzmarkierung.

Die nach diesen Methoden hergestellten, fluoreszierenden Nukleinsäuren können als Gensonden zum Nachweis von Nukleinsäuren bestimmter Sequenz durch Hybridisierung verwendet werden. Die mit Fluoreszenzfarbstoffen markierten Gensonden können in allen bekannten Hybridisierungsassays verwendet werden. Solche Methoden sind aus der Literatur durch zahlreiche Publikationen gut bekannt. Der Nachweis der fluoreszierenden Hybride kann direkt durch Fluoreszenzspektroskopie oder Fluoreszenzmikroskopie erfolgen.

Dieses erfindungsgemäße Verfahren kann besonders vorteilhaft angewendet werden zur in-situ-Hybridisierung mit fixierten ganzen Zellen, mit fixierten Gewebeabstrichen und isolierten Chromosomen sowie zum Nachweis von Virus- und bakteriellen Infektionen in Blut, Serum oder anderen Körperflüssigkeiten.

Ein weiterer Gegenstand dieser Erfindung ist die Verwendung der erfindungsgemäßen cumarinsubstituierten Nukleotide als Enzymsubstrat während eines Amplifikationsverfahrens. Es sind sowohl DNA- als auch RNA-Amplifikationstechniken anwendbar.

Das bekannteste Amplifikationsverfahren ist unter dem Namen "Polymerase Chain Reaction (PCR)" bekannt (EP 2 00 362). Hierbei wird in einer biologischen Probe die nachzuweisende Nukleinsäure in Einzelstränge überführt und mit zwei Oligonukleotiden (Primern) hybridisiert. Beide Oligonukleotide sind zu verschiedenen Teilbereichen des nachzuweisenden Nukleinsäuredoppelstranges komplementär, wobei jeweils ein Oligonukleotid an einen der beiden getrennten Einzelstränge hybridisiert. Nach Hybridisierung wird mit Polymerasen, vorzugsweise mit taq-DNA-Polymerase, und mit Desoxyribonukleotiden als Enzymsubstrat behandelt. Nach Synthese der komplementären DNA, ausgehend von den 3'-OH-Enden der Primer, werden wiederum alle Nukleinsäuredoppelstränge in Einzelstränge überführt und der Vorgang aus Hybridisierung der Primer an die Nukleinsäure und anschließende Polymerisation wiederholt. Durch mehrfache Wiederholung dieser Prozedur kann man die in der Probe enthaltene und nachzuweisende DNA bis zu 10⁹fach vermehren.

Durch Verwendung mindestens eines Desoxyribonukleotides mit einem Cumarinsubstituenten während des Polymerisationsvorganges erhält man amplifizierte Nukleinsäure mit Fluoreszenzmarkierung. Diese Fluoreszenz kann zum Nachweis der Nukleinsäuren herangezogen werden. Das kann nach geeigneter Isolierung der fluoreszenten DNA geschehen, beispielsweise durch Fällung dieser DNA mit EtOH. Weiterhin kann diese Aufarbeitung durch Isolierung der fluoreszenten DNA an komplementäre DNA erfolgen, vorzugsweise an solche DNA, die an Festphasen immobilisiert ist.

Weitere DNA- oder RNA-Amplifikationsmethoden, die die Verwendung der erfindungsgemäßen cumarinsubstituierten Nukleotide erlauben und zur Herstellung von fluoreszenten Amplifikationsprodukten führen, sind z.B. die LCR-(EP-A-320 308), NASBA-(EP-A-329 822), Qβ-(PCT 87/06270) oder HAS-Technik (EP-A-427 074).

Die folgenden Beispiele erläutern die beschriebene Erfindung, sind jedoch in keiner Weise als Einschränkung dieser Erfindung zu verstehen.

### Beispiele

### Beispiel 1

4,0 g des Farbstoffes 7-(N-Ethyl-N-carboxy-trimethylenamino)-3-(4',5'-benzo-thiazo-2'-yl)cumarin werden langsam in 40 ml Oleum (20 %) eingetragen und 2 h bei Raumtemperatur gerührt. Danach wird auf 100 g Eis gegeben und mit konz. Natronlauge auf pH 7 gestellt. Das Wasser wird weitgehend am Rotationsverdampfer abdestilliert und der Rückstand mit 400 ml 10 % Salzsäure 3 h unter Rückfluß erhitzt. Nach Neutralisation mit konz. Natronlauge wird wiederum zur Trockene eingedampft und der feste Rückstand mit 500 ml DMF gerührt. Man filtriert von unlöslichen Salzen ab und entfernt das DMF am Rotationsverdampfer. Nach dem Ausrühren mit 10 bis 20 ml Diethylether erhält man 2,2 g (47 %) des Produkts der Formel 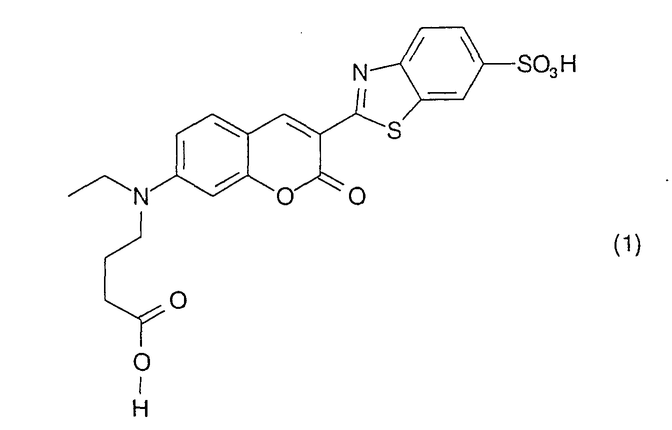 (Na-Salz der Sulfonsäure nach MS-FAB; die Substanz kristallisiert mit einem Mol DMF) Smp: >250°C

### Beispiel 2

Analog erhält man aus 7-(N-Ethyl-N-β-hydroxyethylamino)-3-(4',5'-benzothiazol-2'yl)cumarin in 50 %iger Ausbeute das Produkt der Formel 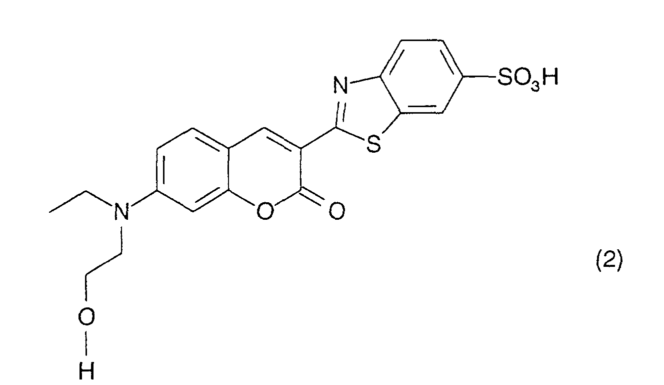 Smp: 270°C (Na-Salz)

### Beispiel 3

Herstellung von 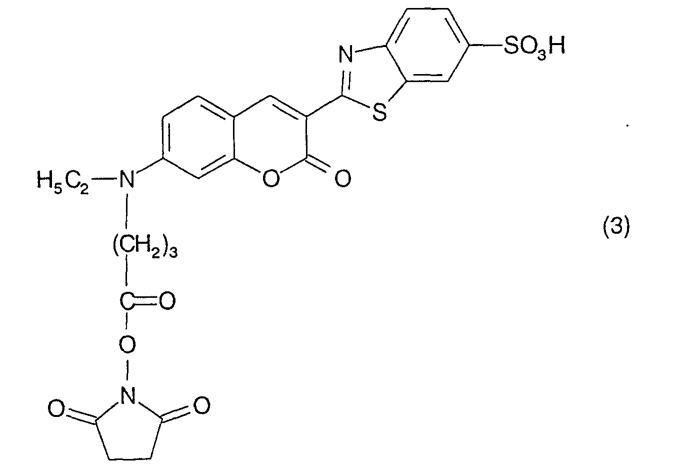 3,0 g (6,1 mmol) der Verbindung (1) der Formel 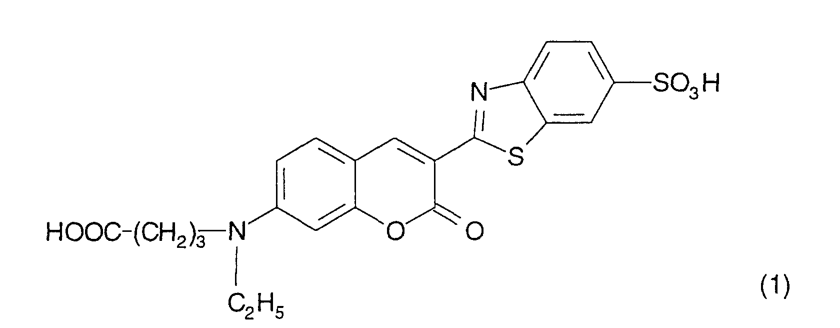 werden in 90 ml Dimethylformamid gelöst. Die Lösung wird auf 50°C erwärmt. Bei dieser Temperatur werden 1,5 ml Pyridin (1,47 g, 18,5 mmol) und 5,7 g Disuccinimidylcarbonat (22,1 mmol) zugegeben. Nach 3 h Rühren bei 50 bis 60°C ist die Umsetzung zum N-Hydroxysuccinimidester erfolgt. Nach Entfernen des Dimethylformamids durch Abdestillieren im Vakuum wird der verbleibende Rückstand säulenchromatographisch gereinigt (Kieselgel, Laufmittel Toluol/Ethanol 1:2). Man erhält 1,8 g eines gelben Feststoffes (50 %) der Formel (3).

### Beispiel 4

Herstellung von 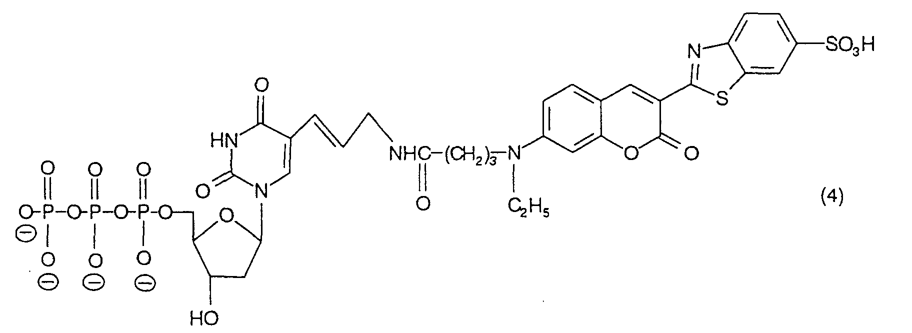 Na₄-Salz
9 mg 5-Allylamino-dUTP (Sigma, 1,6 · 10⁻⁵ mol) werden in 5 ml H₂O gelöst. Die Lösung wird mit 0,2 mol Na₂CO₃ auf pH 9,5 gestellt, danach werden 22 mg des aktivierten Carbonsäureesters der Formel (3) (3,8 · 10⁻⁵ mol) in fester Form zugegeben. Während der Reaktion bei Raumtemperatur wird der pH-Wert durch Zugabe von 0,2 mol Na₂CO₃ zwischen einem Wert von 8,5 bis 9,5 gehalten. Nach Rühren bei Raumtemperatur über Nacht werden innerhalb von 2 h weitere 20 mg (3,4 · 10⁻⁵ mol) des Farbstoffes mit der Formel (3) zugegeben und weiter bei Raumtemperatur gerührt. Nach einer gesamten Laufzeit von 45 h ist dünnschichtchromatographisch (Kieselgel, Ethanol/H₂O 5:2) kein 5-Allylamino-dUTP mehr nachzuweisen. Das Reaktionsgemisch wird im Vakuum bis zur Trockene eingedampft. Das entstandene Reaktionsprodukt wird durch dreimalige Chromatographie an Sephadex ^{R}G 10 (Laufmittel H₂O) gereinigt. Man erhält 3,8 mg des coumarinsubstituierten Deoxyribonukleotids der Formel (4).

### Beispiel 5

Herstellung von 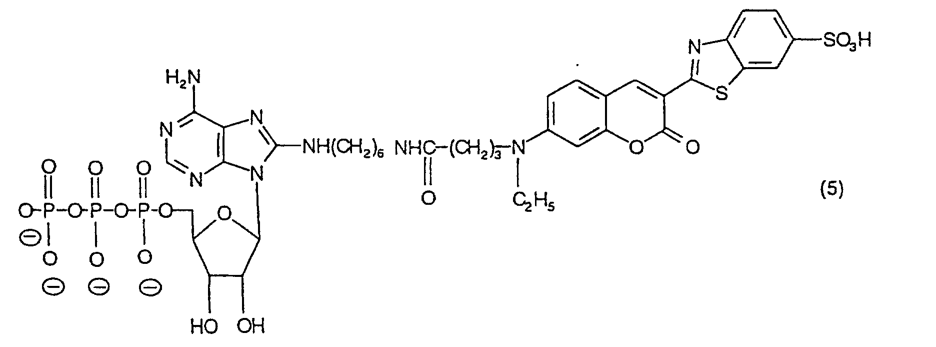 Na₄-Salz

14 mg 8-(6-Aminohexylamino)-ATP (Sigma, 2,26 · 10⁻⁵ mol) werden in 10 ml H₂O gelöst. Die resultierende Lösung wird mit 0,1 mol Na₂CO₃ auf pH 9,5 gestellt. Danach werden bei Raumtemperatur 32 mg des Coumarinderivates der Formel (3) (5,5 · 10⁻⁵ mol) zugegeben und 20 h bei Raumtemperatur gerührt. Der pH-Wert wird durch Zugabe von 0,1 mol Na₂CO₃ im Bereich von 8,5 bis 9.5 gehalten. Dünnschichtchromatographisch (Kieselgel, EtOH/H₂O 5 : 2) ist nach Beendigung der Reaktion kein 8-(6-Aminohexylamino)-ATP mehr nachzuweisen. Das Reaktionsgemisch wird im Vakuum bis zur Trockene eingedampft.

Das entstandene Reaktionsprodukt wird durch dreimalige Chromatographie an Sephadex G 10 (Laufmittel H₂O) gereinigt. Zur Vermeidung von Kontaminationen mit RNAsen werden alle für die Chromatographie benutzten Geräte vor Gebrauch sterilisiert. Man erhält 8,9 mg des fluoreszierenden Nukleotides der Formel (5).

### Beispiel 6

Herstellung von 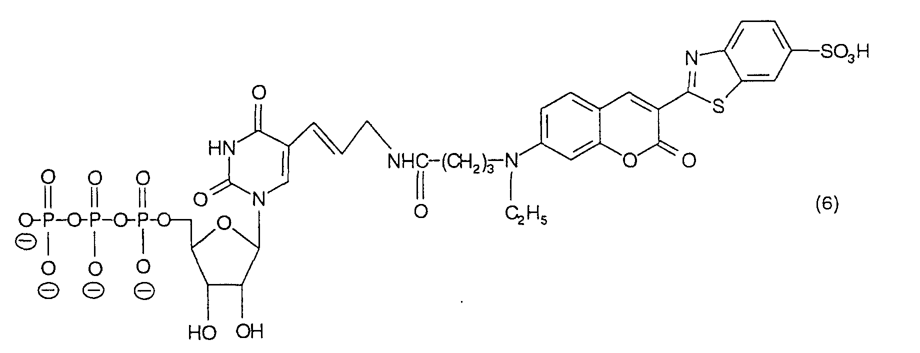 Na₄-Salz

10 mg 5-Allylamino-UTP (1,6 · 10⁻⁵ mol) werden in 5 ml H₂O gelöst Man stellt den pH-Wert durch Zugabe von 0,2 mol Na₂CO₃-Lösung auf pH 9,5 und gibt bei Raumtemperatur 20 mg (3,4 · 10⁻⁵ mol) des Coumarins der Formel (3) zu. Der Ansatz wird 8 h bei Raumtemperatur gerührt, dabei wird der pH-Wert durch Zugabe von 0,2 mol Na₂CO₃-Lösung auf einem Wert von 8,5 bis 9,5 gehalten. Das Reaktionsgemisch wird im Vakuum bis zur Trockene eingedampft. Das entstandene Reaktionsprodukt wird durch dreimalige Chromatographie an Sephadex G 10 (Laufmittel H₂O) gereinigt. Zur Vermeidung von Kontaminationen mit RNAsen werden alle für die Chromatographie benutzten Geräte vor Gebrauch sterilisiert. Man erhält 5,5 mg des fluoreszierenden Nukleotides mit der Formel (6).

### Beispiel 7

Herstellung von 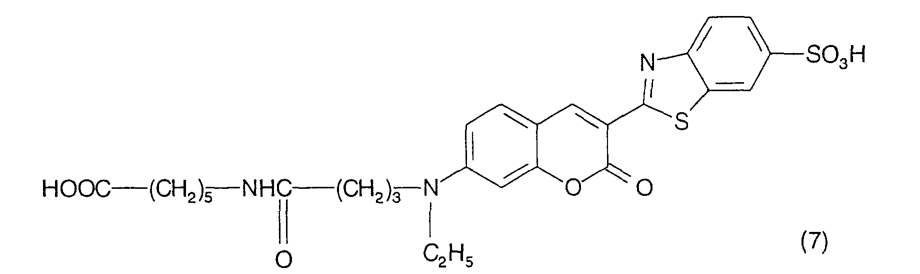 200 mg 6-Aminocapronsäure (1,5 · 10⁻³ mol) werden in 5 ml Wasser gelöst. Der pH-Wert der Lösung wird mit 0,1 mol Na₂CO₃-Lösung auf 9,5 gestellt. Bei Raumtemperatur werden 1,0 g des Carbonsäureesters der Formel (3) (1,7 · 10⁻³ mol) zugegeben und bei Raumtemperatur weitergerührt. Der pH-Wert wird durch Zugabe von 0,1 mol Na₂CO₃-Lösung auf einem Wert von 9 bis 9,5 gehalten. Nach 20 h Rühren ist dünnschichtchromatographisch (Kieselgel, Toluol/Ethanol 1 : 5) das neue Produkt (7) neben wenig verbliebenem Ausgangsmaterial (3) nachzuweisen. Das Reaktionsgemisch wird im Vakuum zur Trockene eingeengt und dann säulenchromatographisch aufgetrennt (Kieselgel, Toluol/Ethanol 1 : 5, danach Ethanol/H₂O 150 : 1).

Die produkthaltigen Fraktionen werden im Vakuum zur Trockene eingeengt, und das verbleibende Produkt mit 50 - 100 ml Diethylether ausgerührt. Man erhält 398 mg (39 %) der Substanz mit der Struktur (7) (Fp 250°C).

### Beispiel 8

Herstellung von 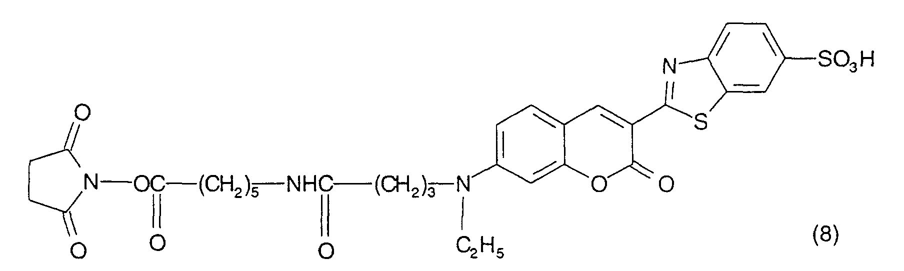 0,35 g der Verbindung (7) (5,8 · 10⁻⁴ mol) und 0,46 g N,N'-Disuccinimidylcarbonat (1,8 · 10⁻³ mol) werden in 3,5 ml DMF gelöst. Die Reaktionsmischung wird 3 h auf 50 bis 60°C erwärmt. Die Lösung wird im Vakuum zur Trockene eingeengt, und das entstandene Produkt (8) wird säulenchromatographisch isoliert (Kieselgel, Toluol/Ethanol 1: 2). Die produkthaltigen Fraktionen werden im Vakuum bis zur Trockene eingeengt und das erhaltene Produkt mit 50 ml Diethylether ausgerührt. Man erhält 248 mg (61 %) der Verbindung (8) [Fp 80°C (Zers.)].

### Beispiel 9

Setzt man 5-Allylamino-dUTP mit der Verbindung (8) um und verfährt, wie im Beispiel 4 für die Umsetzung mit der Verbindung (3) beschrieben, erhält man das fluoreszierende Nukleotid (9) der Formel 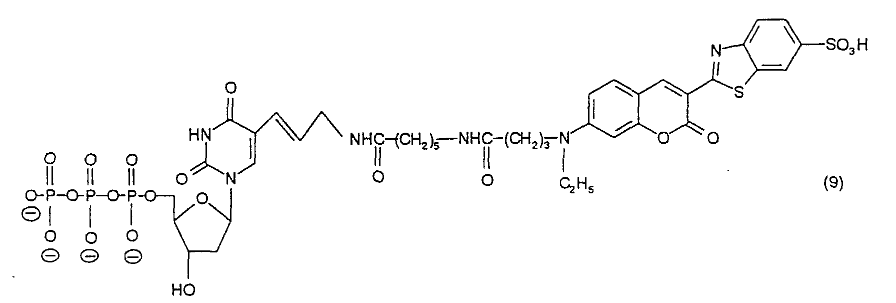 Na₄-Salz

### Beispiel 10

Setzt man 8-(6-Aminohexylamino)-ATP mit der Verbindung (8) um und verfährt. wie im Beispiel 5 für die Umsetzung mit der Verbindung (3) beschrieben, erhält man das fluoreszierende Nukleotid (10) der Formel 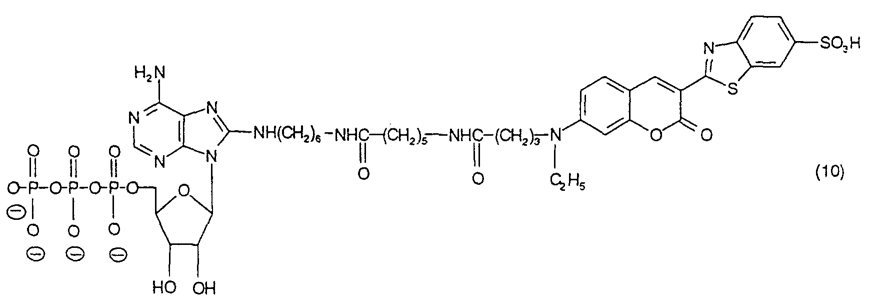 Na₄-Salz

### Beispiel 11

Setzt man 5-Allylamino-UTP mit der Verbindung (8) um und verfährt, wie in Beispiel 6 für die Umsetzung mit der Verbindung (3) beschrieben, erhält man das fluoreszierende Nukleotid (11) der Formel 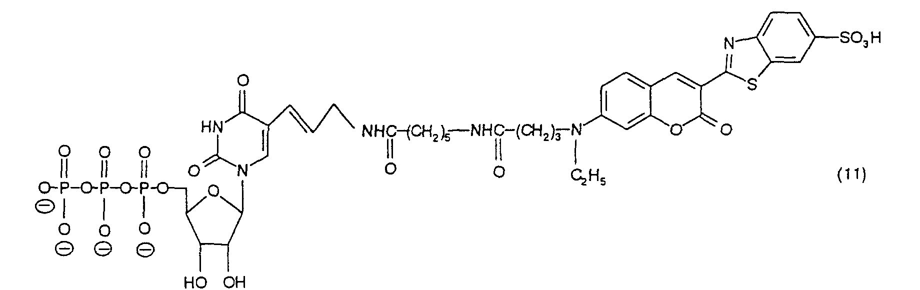 Na₄-Salz

### Beispiel 12

In analoger Weise erhält man durch Verknüpfung der aminosubstituierten Nukleotide mit der Verbindung (2) unter Anwendung geeigneter Hilfsreagentien wie N,N'-Carbonyldiimidazol fluoreszierende Nukleotide, die sich genauso wie die Verbindungen (4) bis (11) für die enzymatische Markierung von Nukleinsäuren einsetzen lassen.

### Beispiel 13

### 3'Endgruppenmarkierung von Polynukleotiden mit fluoreszierenden Nukleotiden.

Eine 1,7 kb lange Polynukleotidsonde wurde mit dem Cumarin-dUTP der Formel (4), Fluoreszein-dUTP, Hydroxy-Cumarin-dUTP und Resorufin-dUTP unter Verwendung eines Endgruppenmarkierungskits der Firma Boehringer Mannheim am 3'Ende markiert. In einem 50 µl Ansatz mit 10 µl Reaktionspuffer (Kaliumkakodylat 1 mol/l; Tris/HCl 125 mmol/l; Rinderserumalbumin 1,25 mg/ml; pH 6,6; 25°C) 1-2 µg Oligonukleotid, 25 Einheiten Terminale Transferase, CoCl₂ 2,5 mmol/l und 0,025 mmol Fluoreszent-dUTP werden nach 60 Minuten bei 37°C ca. 50 % 3'Endmarkierung erreicht.

Die markierten Polynukleotide wurden von den freien NTPs durch geeignete Fällungsmethoden oder säulenchromatographische Methoden abgetrennt und als Gensonden in Slot Blot Hybridisierungen oder Flüssighybridisierungen eingesetzt (Beispiel 18, 19).

Die Fluoreszenz der markierten DNA wurde im Fluoreszenz-Photometer bei der für den einzelnen Cumarinfarbstoff entsprechenden Anregungs- und Emissionswellenlänge gemessen. Es wurde festgestellt, daß die Fluoreszenz der mit dem Cumarin-dUTP der Formel (4) markierten DNA bis um den Faktor 100 höher war als die DNA, die mit Fluoreszein, Hydroxycumarin oder Resorufin markiert worden war.

### Beispiel 14

### Fluoreszenz-Markierung von DNA durch random primed labeling mit Klenow Enzym

Die Methode der random primed DNA-Markierung basiert auf der Hybridisierung einer Mischung von Hexanukleotiden aller möglichen Basensequenzen an die zu markierende DNA. Der komplementäre Strang wird anschließend von den 3' Enden der random Primer mit Hilfe des Klenow Enzyms synthetisiert. Dabei werden die als Substrat angebotenen modifizierten Desoxyribonukleosidtriphosphate, die wie in diesem Beispiel mit Cumarinfarbstoffen markiert sind, in den neu synthetisierten komplementären Strang eingebaut. Bei dem als Primer dienenden Hexanukleotidgemisch sind praktisch alle Sequenzkombinationen enthalten, so daß sie an die eingesetzte DNA in statistischer Verteilung binden und so eine Markierung der gesamten DNA zu gleichen Anteilen garantieren. Die Reaktion ist unabhängig von der Länge der DNA. 200 bp lange Fragmente können ebenso eingesetzt werden wie Polynukleotide mit einer Länge von 5 kb.

Die Verbindung der Formel (4) wird sehr gut in die DNA eingebaut und ergibt im Vergleich zu Fluorescein-dUTP bis zu 100-fach höhere Fluoreszenzsignale.

Mit der random primed DNA-Markierungsmethode wird DNA mit besonders hoher spezifischer Markierung erhalten. Deshalb lassen sich kleinste Mengen markieren. Die so markierte DNA läßt sich bei den verschiedensten Hybridisierungstechniken, wie z.B. in Beispiel 18, 19 beschrieben, verwenden.

### Beispiel 15

### Fluoreszenz-Markierung von DNA durch PCR Amplifikation

Die Polymerase Chain Reaction (PCR) ist in den Patenten EP 200 362 und EP 201 184 beschrieben. Dieses thermozyklische Amplifikationsverfahren basiert auf der Vervielfältigung von DNA-Fragmenten definierter Größe durch die Verwendung von Starteroligonukleotiden, die jeweils durch die Sequenz des Starteroligonucleotids vorgegebene Sequenzbereiche vom 5'Ende mit Hilfe einer thermostabilen Taq-Polymerase komplementär zum Ausgangsstrang synthetisieren. Durch Denaturierung des Doppelstrangs, Annealing der Starteroligonukleotide (Primer) und der Extension neuer DNA-Stränge mit Hilfe der Taq-Polymerase wird durch vielfache Wiederholung dieser Zyklen eine millionenfache Multiplikation des Ausgangs-DNA-Fragmentes erreicht.

Während der Amplifikation können neben den freien Nukleosidtriphosphaten auch Fluoreszenz-NTPs eingesetzt werden und dadurch viele Fluoreszenzmoleküle in die amplifizierte DNA eingebaut werden. Die fluoreszenzmarkierte DNA läßt sich dann direkt mit Hybridisierungsversuchen koppeln und auf diese Weise können hochempfindliche Gensondenteste durchgeführt werden.

Zur PCR-Reaktion wurden eingesetzt: 2 µg genomische DNA von Nitrosomonas europeae, 2 µmol Primer 1 (5'dATCCAGTTGCTTCAAC) und Primer 2 (5'dACTGGCAGGCAGCAG), 2,5 Units Taq-Polymerase von Cetus/Perkin-Elmer sowie jeweils 200 µmol dNTPs in insgesamt 100 µl PCR-Puffer (50 mM KCl, 10 mM Tris/HCl pH 8,3, 1,5 mM MgCl₂ und 0,01 % Gelatine). Von dem Fluoreszenznukleotid der Formel 4 wurde jeweils 40 µl Fluoreszenz-dUTP ergänzt durch 160 µl dTTP eingesetzt. Die Amplifikation wurde in einem PCR-Prozessor der Firma Cetus/Perkin-Elmer durchgeführt.

Mit den Ansätzen wurde zunächst eine Initialschmelzung der DNA 2 Minuten, 30 Sekunden, bei 94°C durchgeführt, dann pro Zyklus 1 Minute bei 94°C die DNA denaturiert, 2 Minuten bei 40 bis 45°C das Primer-Annealing und 3 Minuten bei 72°C die Primer-Extension durchgeführt. Nach 35 Zyklen wurde abschließend eine 20 Minuten-Extension bei 72°C durchgeführt und die Ansätze bei 4°C gekühlt. Die mit der Verbindung (4) markierte amplifizierte DNA ergibt bis zu 100-fach stärkere Fluoreszenzsignale als die mit Fluorescein-dUTP markierte amplifizierte DNA.

### Beispiel 16

### RNA-Markierung durch in vitro Transkription mit fluoreszierenden Ribonukleotiden und RNA Polymerase

Linearisierte Template DNA mit T7 oder T3 Promotor wird unter Verwendung von ATP, GTP, CTP und UTP bzw. fluoreszierenden NTPs mit den entsprechenden RNA-Polymerasen in vitro in RNA transkribiert. Die auf diese Weise markierte RNA kann in Hybridisierungstesten und RNA-Amplifikationstesten wie auch bei der in situ Hybridisierung eingesetzt werden. Die Auswertung erfolgt direkt über die Messung der Fluoreszenz der markierten RNA.

Zur RNA-Transkription und Fluoreszenz wurde ein pSK Bluescript Konstrukt mit T7 Promotor und einem 1,7 kb DNA-Insert eingesetzt. Es wurde folgender Transkriptionsansatz durchgeführt:
10 µl linearisiertes DNA-Konstrukt (0,5 µg)
4 µl Transkriptionspuffer (40 mM Tris/HCl pH 7,9; 6 mM MgCl₂; 10 mM NaCl, 2mM Spermidin,
2 µl 100 mM Dithioerythritol
1 µl10 mM ATP
1 µl10 mM CTP
1 µl10 mM GTP
1 µl0 mM UTP
0,5 µlT 7 Polymerase (10 Einheiten)

Zur Markierung der RNA mit Fluoreszenz-Nukleotiden wurden anstelle von UTP 10 mM der Verbindung der Formel (5) eingesetzt.

Der Ansatz wird 2 Stunden bei 37°C inkubiert, anschließend eine Ethanolfällung durchgeführt und fünfmal mit Ethanol gewaschen und dann die RNA in TE-Puffer wieder gelöst. Die Fluoreszenz wurde im Fluoreszenz-Photometer bestimmt.

### Beispiel 17

### Fluoreszenz-Markierung von RNA durch T7/T3 Hairpin-Amplifikation

Die Hairpin-Amplifikation ist eine RNA-Amplifikationsmethode, die in dem Patent EP 427 074 beschrieben ist. Unter Verwendung eines Hairpin-Oligonukleotids, das einen T7- oder T3 Promotor zur Transkription von RNA mit der entsprechenden RNA-Polymerase ausbildet, besteht die Möglichkeit durch Verwendung von Fluoreszenz-NTPs die RNA zu markieren und millionenfach zu amplifizieren. Durch die Verwendung eines Fluoreszenz-Labels ist eine direkte Auswertung der Amplifikation über das Fluoreszenzsignal im Photometer möglich und es werden keine aufwendigen ELISA-Verfahren notwendig.

Der Transkriptionsansatz wurde, wie in Beispiel 16 angegeben, durchgeführt. Als Fluoreszenznukleotid wurde die Verbindung der Formel (6) eingesetzt. Die Fluoreszenz der markierten Oligonukleotidtranskripte wurde durch Polyacrylamid-Gelelektrophorese ausgewertet.

### Beispiel 18

### Slot Blot Hybridisierung mit fluoreszenzmarkierten DNA oder RNA-Oligonukleotid- oder Polynukleotidsonden

Die Hybridisierung wurde nach üblichen Verfahren durchgeführt bei einer Inkubationstemperatur von 40 bis 68°C. Je nach Hybridisierungstemperatur wurden unterschiedliche Substanzen zugesetzt. Dextransulfat oder andere Polymere wurden eingesetzt, um die Geschwindigkeit und das Ausmaß der Hybridisierung zu erhöhen. Detergentien und Blockierungs-Reagentien wie Trockenmilch, Denhardt's Lösung, Heparin oder SDS wurden zugesetzt, um die unspezifische Bindung der DNA an die Membran zu unterdrücken. Denaturierende Agentien wie Harnstoff oder Formamid können eingesetzt werden, um die Schmelztemperatur der Hybride zu reduzieren, so daß niedrigere Hybridisierungstemperaturen angewandt werden können. Darüber hinaus können durch Zugabe von heterologer DNA die nicht spezifische Bindung von Gensonden an nicht homologer DNA auf dem Blot reduziert werden.

Zur Vorbereitung der Hybridisierung wurden 50 bis 500 ng der nicht markierten genomischen DNA von Nitrosomonas europeae zunächst 5 Minuten bei 100°C denaturiert, auf 0°C abgekühlt und dann auf vorbehandelte Nitrozellulose oder Nylonmembranen mit Hilfe eines Minifoldli^{R}-Filtrationsgeräte der Firma Schleicher und Schüll aufgetragen und bei 80°C 2 Stunden fixiert.

Die Filter wurden in einer versiegelten Plastik-Folientasche oder Plastikbox mit mindestens 20 ml Hybridisierungslösung pro 100 cm² Filter bei 68°C mindestens 1 Stunde hybridisiert.

Die Lösung wurde durch 2,5 ml/100 cm² Filter Hybridisierungslösung ersetzt, der 100 ng Fluoreszenz-Gensonde zugesetzt wurden. Die Filter wurden mindestens 6 Stunden bei 68°C inkubiert unter schwachem Schütteln.

Die Filter wurden dann zweimal 5 Minuten bei Zimmertemperatur mit mindestens 50 ml 2 x SSC, 0,1 % SDS pro 100 cm² Filter und zweimal 15 Minuten bei 68°C mit 0,1 x SSC, 0,1 % SDS gewaschen.

Die Filter wurden dann direkt zur Detektion der hybridisierten DNA eingesetzt.

Lösungen:
- 20 x SSC:: 3M NaCl, 0,3 M Na-Citrat pH 7,0
- Hybridisierungslösung:: 5 x SSC; 0,1 % N-Lauroylsarcosin, Na-Salz, 0,02 % SDS; 0,5 % Blocking Reagenz (Boehringer) Lösung bei 50 bis 70°C lösen.

Andere Hybridisierungslösungen, die ebenfalls für die Slot Blot Hybridisierung eingesetzt werden können, sind z.B.:
Hybridisierungsmix 2: 50 % Formamid; 7 x SSC: 2 x Denhardt's Lösung (100 x Den- hardt's: 2 % Ficoll, 2 % Polyvinylpyrrolidon, 2 % Rinderserumalbumin) 300 µg/ml Kalbsthymus-DNA
Hybridisierungsmix 3: 6 x SSC; 10 x Denhard's Lösung; 50 µg Heringssperma DNA, Rinderserumalbumin 0.1 %
Hybridisierungsmix 4: 5 x SSC; 5 % PEG; % Trockenmilchpulver 0,01 M Natrium pyrophosphat.

Der Read Out erfolgte über den in der Sonde gebundenen Cumarin-Fluoreszenzfarbstoff. Die fluoreszierenden Slot Blots des Filters wurden in einem Shimadzu CS 930 Scanner quantitativ ausgewertet.

### Beispiel 19

### Flüssighybridisierung mit den fluoreszenzmarkierten Oligonukleotid- oder Polynukleotid-Sonden

Flüssighybridisierungen wurden als Sandwich Hybridisierungen mit Streptavidin gecoateten magnetischen Partikeln der Firma Dynal zur Separation des Hybridisierungskomplexes durchgeführt.

Die Flüssighybridisierungsteste wurden als Sandwich Teste mit 100 ng 5'biotinylierter Capture Oligonukleotidsonde mit der Nukleotidsequenz 5'dCTGCTCGTAGACAATGCGT, 100 ng fluoreszenzmarkierter Oligonukleotidsonde (Detector Gensonde) mit der Nukleotidsequenz 5'dATCCAGTTGTGTCTTCAAC und Nitrosomonas Target-DNA in verschiedenen Konzentrationen (50 ng bis 1000 ng) in einem Volumen von 50 µl durchgeführt.

Nach 10-minütigem Erhitzen auf 100°C und anschließendem Abkühlen auf 0°C wurden 50 µl 2 x Boehringer Hybridisierungsmix zugegeben und 1 Stunde bei 68°C hybridisiert. Die magnetischen Beads wurden mit 1 x Boehringermix vorbehandelt und nach dem Separieren über einen Magneten die Flüssigkeit abpipettiert und der Hybridisierungsansatz zugegeben und 1/2 Stunde bei Raumtemperatur unter schwacher Bewegung inkubiert. Der gekoppelte Hybridisierungskomplex wurde mit den Beads separiert, die Restflüssigkeit abpipettiert und einmal mit Puffer A (2 x SSC; 0,1 % SDS)', dann mit Puffer B (0,1 SSC; 0,1 % SDS) zweimal gewaschen. Die magnetischen Partikel wurden in 200 µl SauIIIa Restriktionsenzym-Puffer aufgenommen und nach Zugabe von 2 Units SauIIIa Restriktionsenzym 1 Stunde bei 37°C die fluoreszenzmarkierte DNA von den Beads abgespalten.

Anschließend wurden 400 µl bidest H₂O zugegeben und die Fluoreszenz der DNA in einem Fluoreszenzphotometer gemessen.

### Beispiel 20

### Flüssighybridisierung mit amplifizierter fluoreszenzmarkierter Target-DNA

Die Flüssighybridisierungen wurden als Reversed Phase Teste durchgeführt. Eine Nitrosomonas spezifische 1,7 kb Gensonde wurde dazu am 3'Ende mit einem 3'Endgruppen-Labeling Kit der Firma Boehringer biotinyliert und dann wie im Beispiel 19 mit der fluoreszenzmarkierten amplifizierten genomischen DNA von Nitrosomonas hybridisiert. Der fluoreszierende Hybridisierungskomplex wurde über die biotinylierte Gensonde an Streptavidin gecoateten magnetischen Partikeln aus dem Gesamtansatz abgetrennt und nach entsprechenden Waschschritten wie im Beispiel 19 mit SauIIIa Restriktionsenzym die fluoreszierende DNA von den magnetischen Partikeln abgespalten und im Fluoreszenz-Photometer gemessen.

## Patentansprüche

1. Fluoreszierende Nukleotide der Formel
A - B - C
wobei
A ein natürliches oder synthetisches Nukleotid oder ein Derivat hiervon,
B ein Brückenglied mit zwei verknüpfbaren Zentren
und
C einen Cumarinrest der Formel 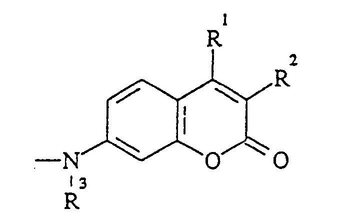 bedeutet, wobei
R¹ Wasserstoff oder Cyano ist,
R² Phenyl oder Thiazolyl, das in 2-, 4- oder 5-Position gebunden ist, bedeutet, wobei beide Reste einen SO₃H-Rest tragen und gegebenenfalls zusätzlich substituiert sind,
oder wobei der Thiazolylrest in 4- oder 5-Position mit einem Benzolring kondensiert ist, der mit einer Sulfogruppe substituiert ist und gegebenenfalls zusätzlich substituiert ist,
R³ H, C₁₋₄-Alkyl, C₁₋₄-Alkoxycarbonyl-C₁₋₄-alkyl, -alkyl, oder Phenyl- sulfonyl bedeutet, wobei C₁₋₄-Alkyl unsubstituiert oder mit Hydroxyl, Amino, Carboxyl, C₁₋₄-Alkoxycarbonyl oder Sulfo substituiert sein kann, und wobei Phenylsulfonyl unsubstituiert oder einfach oder mehrfach durch Chlor, Brom. C₁₋₄-Alkyl oder Sulfo substituiert sein kann.

2. Fluoreszierende Nukleotide gemäß Anspruch 1, **dadurch gekennzeichnet, daß**
A einen Rest darstellt, der aus der Gruppe von natürlichen oder synthetischen Nukleotiden ausgewählt wird, die aus AMP, ADP, ATP, GMP, GDP, GTP, CMP, CDP, CTP, UMP, UDP, UTP, TMP, TDP, TTP, 2-Me-AMP, 2-Me-ADP, 2-Me-ATP, 1-Me-GMP, 1-Me-GDP, 1-Me-GTP, 5-Me-CMP, 5-Me-CDP, 5-MeO-CTP, 5-MeO-CMP, 5-MeO-CDP, 5-Me-CTP, Deoxy- oder Dideoxynukleotide aus dieser Reihe, sowie weitere Derivate hiervon, besteht,
B ein bifunktionelles Brückenglied mit bis zu 50 Atomen Kettenlänge ist, wobei die Atome C, H, O, N oder S sein können und das Brückenglied linear oder verzweigt sein kann,
und
C einen Cumarinrest der Formel 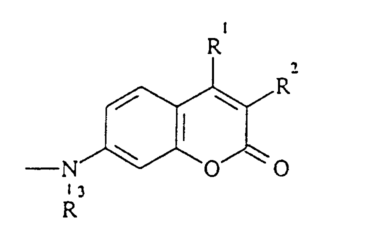 darstellt, in dem
R¹ Wasserstoff oder Cyano bedeutet,
R² Phenyl oder Thiazolyl, das 2-, 4- oder 5-Position gebunden ist, bedeutet, wobei Phenyl mit einer Sulfogruppe substituiert ist und zusätzlich mit Carboxy, C₁₋₄-Alkylcarbonyloxy, Amino, -NH-C₁₋₄-Alkyl, -(CH₂)₁₋₄-NH₂, C₁₋₄-Alkyl, Cyano, Fluor, Chlor, Brom oder Sulfo substituiert ist und wobei der Thiazolylrest mit einer Sulfogruppe substituiert ist und gegebenenfalls noch zusätzliche Substituenten tragen kann oder wobei der Thiazolylrest in 4- und 5-Position mit einem Benzolring kondensiert ist, der mit einer Sulfogruppe substituiert ist sowie zusätzlich substituiert sein kann,
und
R³ Wasserstoff, Methyl, Ethyl, -(CH₂)₁₋₄-OH, -(CH₂)₁₋₄-NH₂ oder -(CH₂)₁₋₄-COOH oder (CH₂)₁₋₄-SO₃H bedeutet.

3. Fluoreszierende Nukleotide gemäß Anspruch 1 und 2, **dadurch gekennzeichnet, daß**
A einen Rest darstellt, der aus der Gruppe von natürlichen oder synthetischen Nukleotiden ausgewählt wird, die aus AMP, ADP, ATP, GMP, GDP, GTP, CMP, CDP, CTP, UMP, UDP, UTP, TMP, TDP, TTP, 2-Me-AMP, 2-Me-ADP, 2-Me-ATP, 1-Me-GMP, 1-Me-GDP, 1-Me-GTP, 5-Me-CMP, 5-Me-CDP, 5-MeO-CTP, 5-MeO-CMP, 5-MeO-CDP, 5-Me-CTP, Deoxy- oder Dideoxynukleotide aus dieser Reihe, sowie weitere Derivate hiervon, besteht,
und
B ein bifunktionelles Brückenglied mit 2 bis 20 Atomen aus der Reihe von C, H. O, N oder S ist.
und
C einen Cumarinrest der Formel 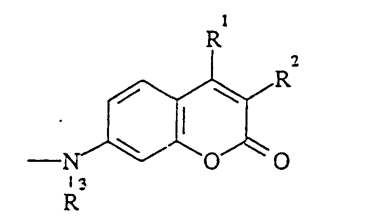 bedeutet, wobei
R¹ Wasserstoff oder Cyano bedeutet,
R² Phenyl oder Thiazolyl, das in 2-Position gebunden ist, bedeutet
wobei Phenyl mit einer Sulfogruppe substituiert ist und zusätzlich mit para-Carboxy, para-Amino, para-NH-C₁₋₄-Alkyl, para-CH₂-NH₂, Cyano, Methyl oder Ethyl substituiert ist, und wobei Thiazolyl mit Sulfo substituiert ist und zusätzlich mit Chlor, Cyano oder Carboxyl substituiert ist, oder der Thiazolylrest in 4- und 5-Stellung mit einem Benzolring kondensiert ist, der mit Sulfo substituiert ist und zusätzlich Carboxy oder Amino als Substituenten enthalten kann.
und
R³ Wasserstoff, Methyl, Ethyl, -(CH₂)₁₋₄-OH, -(CH₂)₁₋₄-NH₂ oder -(CH₂)₁₋₄-COOH oder (CH₂)₁₋₄-SO₃H bedeutet.

4. Verwendung der Verbindungen nach einem der Ansprüche 1 bis 3 zum enzymatischen Einbau in Nukleinsäuren, wobei die verwendeten Enzyme aus der Gruppe der DNA-Polymerasen, RNA-Polymerasen, Reversen Transkriptasen oder Terminalen Transferasen bestehen.

5. Verwendung der Nukleoside nach den Ansprüchen 1 bis 3, im Rahmen von Amplifikationsverfahren zum Nachweis von Nukleinsäuren.

6. Gensonden enthaltend ein oder mehrere der Nukleotide gemäß den Ansprüchen 1 bis 3.

7. Reagenz zum Nachweis von Nukleinsäuren enthaltend eines oder mehrere der Nukleotide gemäß den Ansprüchen 1 bis 3.

## Claims

1. Fluorescent nucleotides of the formula
A - B - C
where
A denotes a natural or synthetic nucleotide or a derivative thereof,
B denotes a bridging member having two linkable centres,
and
C denotes a coumarin residue of the formula 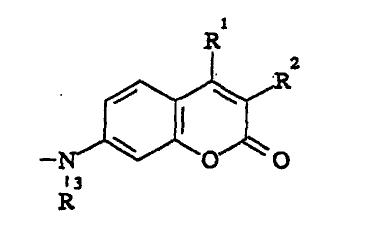
where
R¹ is hydrogen or cyano,
R² is phenyl or thiazolyl, which is bound in the 2, 4 or 5 position, where both residues carry a SO₃H radical and can be additionally substituted, or where the thiazolyl residue is condensed in the 4 and 5 positions with a benzene ring which is substituted by a sulpho group and can be additionally substituted,
R³ is H, C₁₋₄-alkyl, C₁₋₄-alkoxycarbonyl-C₁₋₄-alkyl, or phenylsulphonyl, where C₁₋₄-alkyl can be unsubstituted or substituted by hydroxyl, amino, carboxyl, C₁₋₄-alkoxycarbonyl or sulpho, and where phenylsulphonyl can be unsubstituted or substituted once or more than once by chlorine, bromine, C₁₋₄-alkyl or sulpho.

2. Fluorescent nucleotides according to Claim 1, **characterized in that**
A represents a residue which is selected from the group of natural or synthetic nucleotides consisting of AMP, ADP, ATP, GMP, GDP, GTP, CMP, CDP, CTP, UMP, UDP, UTP, TMP, TDP, TTP, 2-Me-AMP, 2-Me-ADP, 2-Me-ATP, 1-Me-GMP, 1-Me-GDP, 1-Me-GTP, 5-Me-CMP, 5-Me-CDP, 5-Me-CTP, 5-MeO-CMP, 5-MeO-CDP, 5-MeO-CTP, and deoxynucleotides or dideoxynucleotides from this series, as well as further derivatives thereof,
B is a bifunctional bridging member having a chain length of up to 50 atoms, where the atoms can be C, H, O, N or S and the bridging member can be linear or branched,
and
C represents a coumarin residue of the formula 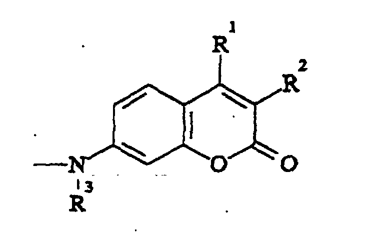 in which
R¹ is hydrogen or cyano,
R² is phenyl or thiazolyl, which is bound in the 2, 4 or 5 position, where phenyl is substituted by a sulpho group and is additionally substituted by carboxyl, C₁₋₄-alkylcarbonyloxy, amino, -NH-C₁₋₄-alkyl, - (CH₂)₁₋₄-NH₂, C₁₋₄-alkyl, cyano, fluorine, chlorine, bromine or sulpho and where the thiazolyl residue is substituted by a sulpho group and can optionally carry additional substituents, or where the thiazolyl residue is condensed in the 4 and 5 positions with a benzene ring which is substituted by a sulpho group and can be additionally substituted,
and
R³ is hydrogen, methyl, ethyl, -(CH₂)₁₋₄-OH, - (CH₂)₁₋₄-NH₂ or -(CH₂)₁₋₄-COOH or (CH₂)₁₋₄-SO₃H.

3. Fluorescent nucleotides according to Claims 1 and 2, **characterized in that**
A represents a residue which is selected from the group of natural or synthetic nucleotides consisting of AMP, ADP, ATP, GMP, GDP, GTP, CMP, CDP, CTP, UMP, UDP, UTP, TMP, TDP, TTP, 2-Me-AMP, 2-Me-ADP, 2-Me-ATP, 1-Me-GMP, 1-Me-GDP, 1-Me-GTP, 5-Me-CMP, 5-Me-CDP, 5-Me-CTP, 5-MeO-CMP, 5-MeO-CDP, 5-MeO-CTP, and deoxynucleotides or dideoxynucleotides from this series, as well as further derivatives thereof,
and
B is a bifunctional bridging member having 2 to 20 atoms selected from the group comprising C, H, O, N and S,
and
C is a coumarin residue of the formula 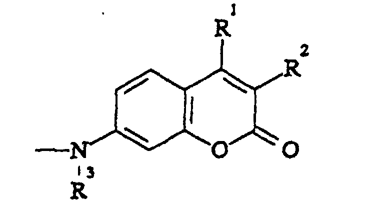 where
R¹ is hydrogen or cyano,
R² is a phenyl or thiazolyl, which is bound in the 2 position,
where phenyl is substituted by a sulpho group and is additionally substituted by para-carboxyl, para-amino, para-NH-C₁₋₄-alkyl, para-CH₂-NH₂, cyano, methyl or ethyl, and where thiazolyl is substituted by sulpho and is additionally substituted by chloro, cyano or carboxyl, or the thiazolyl residue is condensed in the 4 and 5 positions with a benzene ring which is substituted by sulpho and can additionally contain carboxyl or amino as substituent,
and
R³ is hydrogen, methyl, ethyl, -(CH₂)₁₋₄-OH, - (CH₂)₁₋₄-NH₂ or -(CH₂)₁₋₄-COOH or (CH₂)₁₋₄-SO₃H.

4. Use of the compounds according to one of Claims 1 to 3 for enzymic incorporation into nucleic acids, the enzymes used consisting of the group of the DNA polymerases, RNA polymerases, reverse transcriptases or terminal transferases.

5. Use of the nucleosides according to Claims 1 to 3 in amplification processes for detecting nucleic acids.

6. Gene probes containing one or more of the nucleotides according to Claims 1 to 3.

7. Reagent for detecting nucleic acids containing one or more of the nucleotides according to Claims 1 to 3.

## Revendications

1. Nucléotides fluorescents de formule
A-B-C
dans laquelle
A est un nucléotide naturel ou synthétique ou un dérivé de ce nucléotide,
B est un chaînon de pontage ayant deux centres susceptibles de liaison
et
C représente un reste de coumarine de formule 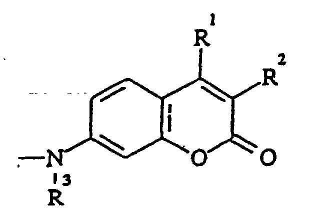 dans laquelle
R¹ est l'hydrogène ou le groupe cyano,
R² représente un groupe phényle ou thiazolyle qui est lié en position 2, 4 ou 5, les deux restes portant un reste SO₃H et présentant, le cas échéant, une autre substitution,
ou bien le reste thiazolyle est condensé en position 4 ou 5 avec un noyau benzénique qui est substitué avec un groupe sulfo et présente éventuellement une autre substitution,
R³ représente H, un groupe alkyle en C₁ à C₄, (alkoxy en C₁ à C₄) carbonyl-(alkyle en C₁ à C₄) ou un groupe phénylsulfonyle, le groupe alkyle en C₁ à C₄ étant non substitué ou pouvant être substitué avec un radical hydroxyle, amino, carboxyle, (alkoxy en C₁ à C₄)carbonyle ou sulfo et le groupe phénylsulfonyle étant non substitué
ou pouvant être substitué une ou plusieurs fois par un radical chloro, bromo, alkyle en C₁ à C₄ ou sulfo.

2. Nucléotides fluorescents suivant la revendication 1, **caractérisés en ce que**
A représente un reste qui est choisi dans le groupe de nucléotides naturels ou synthétiques, qui consiste en AMP, ADP, ATP, GMP, GDP, GTP, CMP, CDP, CTP, UMP, UDP, UTP, TMP, TDP, TTP, 2-Me-AMP, 2-Me-ADP, 2-Me-ATP, 1-Me-GMP, 1-Me-GDP, 1-Me-GTP, 5-Me-CMP, 5-Me-CDP, 5-MeO-CPT, 5-MeO-CMP, 5-MeO-CDP, 5-Me-CTP, désoxy- ou didésoxynucléotides de cette série, ainsi que d'autres dérivés de ces nucléotides,
B est un chaînon bifonctionnel de pontage de longueur de chaîne allant jusqu'à 50 atomes, les atomes pouvant être des atomes C, H, O, N ou S et le chaînon de pontage pouvant être linéaire ou ramifié,
et
C représente un reste de coumarine de formule 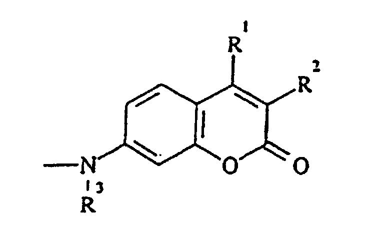
dans laquelle
R¹ est l'hydrogène ou le groupe cyano,
R² représente un reste phényle ou thiazolyle qui est lié en position 2, 4 ou 5, le reste phényle étant substitué avec un groupe sulfo et substitué en outre avec un groupe carboxy, (alkyle en C₁ à C₄)-carbonyloxy, amino, -NH-(alkyle en C₁ à C₄)- (CH₂)₁₋₄-NH₂, alkyle en C₁ à C₄, cyano, fluoro, chloro, bromo ou sulfo et le reste thiazolyle étant substitué avec un groupe sulfo et pouvant porter, le cas échéant, encore d'autres substituants
ou bien le reste thiazolyle est condensé en positions 4 et 5 avec un noyau benzénique qui est substitué avec un groupe sulfo et qui peut même présenter une autre substitution
et
R³ représente l'hydrogène, un groupe méthyle, éthyle, -(CH₂)₁₋₄-OH, -(CH₂)₁₋₄-NH₂ ou - (CH₂)₁₋₄-COOH ou - (CH₂)₁₋₄-SO₃H.

3. Nucléotides fluorescents suivant les revendications 1 et 2, **caractérisés en ce que**
A représente un reste qui est choisi dans le groupe de nucléotides naturels ou synthétiques, qui consiste en AMP, ADP, ATP, GMP, GDP, GTP, CMP, CDP, CTP, UMP, UDP, UTP, TMP, TDP, TTP, 2-Me-AMP, 2-Me-ADP, 2-Me-ATP, 1-Me-GMP, 1-Me-GDP, 1-Me-GTP, 5-Me-CMP, 5-Me-CDP, 5-MeO-CPT, 5-MeO-CMP, 5-MeO-CDP, 5-Me-CTP, désoxy- ou didésoxynucléotides de cette série, ainsi que d'autres dérivés de ces nucléotides,
et
B est un chaînon bifonctionnel de pontage ayant 2 à 20 atomes de la série C, H, O, N ou S,
et
C représente un reste de coumarine de formule 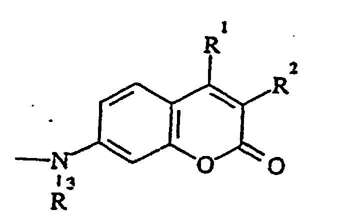 dans laquelle
R¹ représente l'hydrogène ou le groupe cyano,
R² représente un reste phényle ou thiazolyle qui est lié en position 2, le reste phényle étant substitué avec un groupe sulfo et substitué en outre avec un groupe paracarboxy, para-amino, para-NH-(alkyle en C₁ à C₄), para-CH₂-NH₂, cyano, méthyle ou éthyle et le reste thiazolyle étant substitué avec un groupe sulfo et substitué en outre avec un radical chloro, cyano ou carboxyle, ou bien le reste thiazolyle est condensé en positions 4 et 5 avec un noyau benzénique qui porte un substituant sulfo et qui peut porter en outre des groupes carboxy ou amino comme substituants,
et
R³ représente l'hydrogène, un groupe méthyle, éthyle, -(CH₂)₁₋₄-OH, -(CH₂)₁₋₄-NH₂ ou -(CH₂)₁₋₄-COOH ou -(CH₂)₁₋₄-SO₃H.

4. Utilisation des composés suivant l'une des revendications 1 à 3 pour l'incorporation enzymatique dans des acides nucléiques, les enzymes utilisées consistant en enzymes du groupe des ADN-polymérases, ARN-polymérases, transcriptases inverses ou transférases terminales.

5. Utilisation des nucléotides suivant les revendications 1 à 3 dans le cadre de procédés d'amplification pour la détection d'acides nucléiques.

6. Sondes géniques contenant un ou plusieurs des nucléotides suivant les revendications 1 à 3.

7. Réactif pour la détection d'acides nucléiques, contenant un ou plusieurs des nucléotides suivant les revendications 1 à 3.


### SEQUENZPROTOKOLL

(1) ALLGEMEINE INFORMATION:
   (i) ANMELDER:
      (A) NAME: Bayer AG
      (B) STRASSE: Bayerwerk
      (C) ORT: Leverkusen
      (E) LAND: Deutschland
      (F) POSTLEITZAHL: W-5090
      (G) TELEPHON: 0214/3061455
      (H) TELEFAX: 0214/303482
   (ii) ANMELDETITEL: Sulfocumarinhaltige Nucleotide und deren Verwendung
   (iii) ANZAHL DER SEQUENZEN: 4
   (iv) COMPUTER-LESBARE FORM:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.25 (EPA)
(2) INFORMATION ZU SEQ ID NO: 1:
   (i) SEQUENZ CHARAKTERISTIKA:
      (A) LÄNGE: 16 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: NEIN
   (vi) URSPRÜNLICHE HERKUNFT:
      (B) STAMM: Nitrosomonas europeae
   (xi) SEQUENZBESCHREIBUNG: SEQ ID NO: 1:
   ATCCAGTTGC TTCAAC 16
(2) INFORMATION ZU SEQ ID NO: 2:
   (i) SEQUENZ CHARAKTERISTIKA:
      (A) LÄNGE: 15 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: NEIN
   (vi) URSPRÜNLICHE HERKUNFT:
      (B) STAMM: Nitrosomonas europeae
   (xi) SEQUENZBESCHREIBUNG: SEQ ID NO: 2:
   ACTGGCAGGC AGCAG 15
(2) INFORMATION ZU SEQ ID NO: 3:
   (i) SEQUENZ CHARAKTERISTIKA:
      (A) LÄNGE: 19 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: NEIN
   (vi) URSPRÜNLICHE HERKUNFT:
      (B) STAMM: Nitrosomonas europeae
   (xi) SEQUENZBESCHREIBUNG: SEQ ID NO: 3:
   CTGCTCGTAG ACAATGCGT 19
(2) INFORMATION ZU SEQ ID NO: 4:
   (i) SEQUENZ CHARAKTERISTIKA:
      (A) LÄNGE: 19 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: NEIN
   (vi) URSPRÜNLICHE HERKUNFT:
      (B) STAMM: Nitrosomonas europeae
   (xi) SEQUENZBESCHREIBUNG: SEQ ID NO: 4:
   ATCCAGTTGT GTCTTCAAC 19